Europäisches Patentamt

European Patent Office

Office européen des brevets

(19)

(11) **EP 1 645 288 A1**

(12) **EUROPEAN PATENT APPLICATION**

(43) Date of publication:
**12.04.2006 Bulletin 2006/15**

(21) Application number: **04425751.7**

(22) Date of filing: **07.10.2004**

(51) Int Cl.:
*A61K 47/48* (2006.01)    *A61K 31/385* (2006.01)
*A61K 31/407* (2006.01)    *A61P 35/00* (2006.01)
*A61P 9/00* (2006.01)    *A61P 19/04* (2006.01)
*A61P 25/00* (2006.01)    *A61P 1/00* (2006.01)
*A61P 17/00* (2006.01)    *A61P 31/00* (2006.01)
*A61P 13/00* (2006.01)    *A61P 15/00* (2006.01)

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **CTG Pharma S.r.l.**
**20123 Milano (IT)**

(72) Inventor: **Sparatore, Anna**
**20146 Milan (IT)**

(74) Representative: **Parisi, Luigi**
**Ufficio Internazionale Brevetti,**
**Ing. C. Gregorj S.p.A.**
**Via Dogana 1**
**20123 Milano (IT)**

(54) **New nuclear transcription factors regulators**

(57)    The present invention relates to novel compounds that are nuclear transcription factors (NTF) regulators. The present invention also provides methods for treating, preventing and/or reducing inflammation-associated diseases by regulating NTF in the cardiovascular, connective tissue, pulmonary, gastrointestinal, respiratory, urogenital, nervous, or cutaneous systems as well as tumoral and infective diseases employing said compounds.

The present invention is based on the discovery that it is possible to link regulators of NTF to a pharmacologically active compound helpful for treating disorders in the cardiovascular, connective tissue, pulmonary, gastrointestinal, respiratory, urogenital, nervous, or cutaneous systems or tumoral and infective diseases. The resulting compounds have good bioavailability, increased activity and/or safety.

EP 1 645 288 A1

**Description**

Field of the invention

This invention relates to the field of compounds able to regulate nuclear transcription factors (NTFs). NTFs activation is involved in a variety of human diseases such as atherosclerosis, asthma, arthritis, cancer, diabetes, AIDS, inflammatory bowel diseases and stroke. The development of NTF regulators should reduce side effects associated with drugs such as, for example, NSAIDs, glucocorticoids etc.

Background of the invention

Many NTFs have been described in the literature. A non exclusive list of factors that are involved in the inflammatory process include nuclear factor kappa B (NF-kB), activator protein 1 (AP-1), specificity protein 1 (Sp1), peroxisome proliferator-activated receptors (PPARs), Nr2/small Maf and other members of the nuclear receptor superfamily (K. Kataoka et al. in J. Biol. Chem. 2001, 276 (36), pag. 34074-81 and Y. Lavrovsky et al. in Exp. Gerontol. 2000, 35, pag. 521-32).

Among these NTFs, NF-kB is one of the most studied and will be described as non-limitative example. It was discovered by David Baltimore's group in 1986, ubiquitously expressed that represents a group of five mammalian, structurally related, proteins also called Rel/NF-kB proteins (R. Sen and D. Baltimore in Cell 1986, 47, pag. 921-928). NF-kB plays a central role in regulating expression of a large number of genes that are critical for the regulation of apoptosis, viral replication, tumorigenesis, inflammation etc. Cytokines that are stimulated NF-kB, such as IL-1$\beta$ and TNF-$\alpha$, can also directly activate the NF-kB pathway, thus establishing loops that can amplify the inflammatory response and increase the duration of chronic inflammation. NF-kB also stimulates the expression of enzymes whose products contribute to the pathogenesis of inflammatory process, including the inducible form of nitric oxide synthase (iNOS), which generates nitric oxide (NO), and the inducible cyclooxygenase (COX2), which generates prostanoids (Y. Yamamoto and R. Gaynor in J. Clin. Invest. 2001, 107, pag. 135-142). The activation of NF-kB is thought to be part of a stress response as it is activated by various factors that include growth factor, cytokines, lymphokynes, viral proteins, ischemia/ reperfusion, UV, pharmacological agents, and oxidative. Generally, inflammation has been implicated in a very large number of disorders either as a cause of primary disease process or at least as consequence leading to mild/severe complications. Therefore, there is a need in the art for more effective and safer drugs for inflammation-associated disorders in the cardiovascular system (for example myocardial and vascular ischemia in general, hypertension systemic and regional, stroke, atherosclerosis, etc), connective tissue (for example arthritis and connected inflammatory diseases, etc.), pulmonary ssstem (for example asthma, COPD, etc.), gastrointestinal system (for example ulcerative and non-ulcerative diseases, intestinal inflammatory diseases, liver cirrhosis, etc), urogenital system (for example impotence, incontinence, etc.), central nervous system ( Alzheimer disease, Parkinson's disease and neurogenerative diseases in general), cutaneous system (eczema, neurodermatitis, acne, etc.), infective disease (of bacteria, viruses, parasites origin) and for antineoplastic therapy in different organs (colon, lung, prostate, ovaries, uterus, breast, tongue, liver, bone, etc.), in prevention and/or treatment as single therapy or cotherapy with other chemiotherapic agents or radiotherapic interventions.

Tissue damage by infection, trauma, toxins, abnormally low or high temperatures and other reasons, usually leads to formation of increased amounts of pathogenetic mediators, such as prostaglandins, cytokines, leukotrienes, interleukins, oxy-, thiyl- and nitrogen-free radicals, interacting each other. A serious, outstanding medical need is the development of NTF specific inhibitors that afford an efficacious treatment while minimizing unwanted effects. The research has been focused in the past to develop effective therapeutic agents able to counteract the deleterious effects of such mediators, but results have been so far either unsatisfactory or only partially satisfactory. In fact it has been described (see for example the review by Tak PP and Firestein GS JCI,107,7-11,2001) that some compounds, including corticosteroids, sulfasalazine, 5-aminosalicylic acid, aspirin, NO-releasing aspirin, tepoxalin, leflunomide, curcumin, antioxidants, proteasome inhibitors are able to inhibit NF-kB only at relatively high concentrations. Thus this appears to be the cause for the preclusion of the utilization of NF-kB inhibitors in therapy. An alternative and/or complementary approach is to develop agents able to potentiate the natural defences. In this class of agents a variety of moieties have been described, including again in a non-limitative and exclusive way: heme oxygenase-1, y-glutamylcysteine synthetase, IL10, Mn-SOD, Catalase, DT-diaphorase, glutathione peroxidase, thioredoxin system, NADPH-oxidase, NAG-1, p53.

The ideal approach is to develop compounds able to both counteract aggressive and pathogenetic factors and potentiate defensive substances. This can be obtained by combining chemically a drug known to counteract one or more of the above mentioned pathogenetic mediators with other moieties able to regulate the activation of NTF.

The new compounds object of the present invention, that are able to inhibit the formation of a pathogenetic mediator, such as inducible NOS at non-toxic concentrations, and to stimulate the formation of a defensive agent, such as heme oxygenase-1, have the following chemical structure:

$$D - X - Y - S \qquad (I)$$

wherein D is a drug, X is zero or a chemical functional group, such as C=O, COOR, CONH, R being straight or branched $C_1$-$C_4$-alkyl,
Y is zero or a bifuntional linker, such as HO-$(CH_2)$n-OH, HOOC-$(CH_2)_n$-COOH, and S is a moiety capable per se or in combination with the drug to modulate the NTFs, with the proviso that when both X and Y are zero, the drug (D) and the moiety (S) are linked by an acid-base bond

$$D^{+/-} . S^{-/+}$$

wherein D is an acid (+) or basic (-) drug and S is a basic (-) or acid (+) compound capable to modulate directly and/or indirectly the NTF that is a salt of an acid (drug⁻ ) with a base (NFs⁺ modulator) or a salt of a base (drug⁺ ) with an acid (NFs- modulator).

<u>General</u>

For the screening approach it was decided to test the compounds object of the present invention and fulfilling the structural general criteria previously described for heme oxygenase-1 (OH-1)and inducible nitric oxide synthase (iNOS). The enzyme tests were performed as described by Vicente AM et al. (Br.J.Pharmacol. 2001,133,920-6). The formation of both these two enzymes was associated with the activation of transcription factors, and NF-kB particularly. The following reference compounds were used: indomethacin as example of unselective cyclooxygenase inhibitor, dexamethasone, as example of steroidal glucorticoid agent, N-acetylcysteine as example of anti-oxidant agent, NS-398 as example of COX-2 inhibitor, zileuton, as example of 5-lipooxygenase inhibitor,L-N-methylarginine (L-NAME), as example of NOS inhibitor.

In brief, Raw 264.7 murine macrophage cell line were maintained in a proper culture medium supplemented with 10% fetal bovine serum,2 mM L-glutamine and penicillin/streptomycin. Suspensions of zymosan A from Saccharomyces cerevisiae in saline were used. After incubation with zymosan and/or test compounds for 18 hrs, cells supernatants were collected to measure protein expression, by western blot analysis. After incubation, macrophages were lysed in 100 microliters of buffer (1% Triton X-100,1% deoxicholic acid,20 mM NaCl and 25 mM Tris,pH 7.4) and then centrifuged at 4°C for 5 min at 10,000 x g. The protein content was determined by the Bradford method using bovine serum albumin as standard. Cell lysate (40 micrograms of protein) was mixed with Laemmli sample buffer under reducing conditions. Samples were sized-separated in 12.5% SDS-PAGE and transferred into polyvinylidene membranes, which were blocked in phosphate buffer saline (0.02 M,pH 7.0)-Tween 20 (0.1%) containing 3% fat-free dry milk. Membranes were incubated with specific antibodies: polyclonal antibody against iNOS (1/1000,Cayman Chemical) and anti-OH-1 monoclonal antibody (1/2000,Stressgen). Blots were washed and incubated with peroxidase-conjugated goat IgG (1/20,000). The immunoreactive bands were visualized by an enhanced chemiluminescence system and band intensity quantitated using computer-assisted densitometry.

Cell viability was assessed by the mitochondrial-dependent reduction of 3-(4,5-dimethyltiazol-2-yl)-2.5 diphenyltetrazolium bromide (MTT) to formazan. After appropriate stimulation times, cells were incubated with MTT (200 micrograms/ml) for 60 min. The medium was then removed and cells were solubilized in dimethylsulphoxide to quantitate formazan at 550 nm.

<u>Results</u>

The results on OH-1 and iNOS were shown in table I. Only the test compound ATANPE was able to stimulate OH-1 production and inhibit iNOS formation. All the compounds did not affect cell integrity and viability under the experimental conditions above described and at the concentrations used.

Table I Effect of some compounds on zymosan-induced OH-1 and iNOS protein expression. Band intensity was calculated as percentage with respect to zymosan.

| Treatment | Concentration ($\mu$mol) | OH-1 | INOS |
|---|---|---|---|
| Basal | ------ | 3 | 2 |

Table continued

| Treatment | Concentration ($\mu$mol) | OH-1 | INOS |
|---|---|---|---|
| Zymosan | ------ | 100 | 100 |
| Dexamethasone | 1 | 20 | 85 |
| Indomethacin | 1 | 65 | 78 |
| NS398 | 10 | 72 | 120 |
| N-acetylcysteine | 100 | 44 | 80 |
| Zileuton | 10 | 125 | 112 |
| L-NAME | 1000 | 70 | 110 |
| ATANPE | 10 | 293 | 48 |

It has been found that the compounds fulfilling these criteria surprisingly showed not only an efficacy greatly increased, with additive or synergistic implementation, but also a safety profile dramatically improved.

## SUMMARY OF THE INVENTION

The present invention is based on the discovery that it is possible to link regulators of NTF to a pharmacologically active compound helpful for treating disorders in the cardiovascular, connective tissue, pulmonary, gastrointestinal, respiratory, urogenital, nervous, or cutaneous systems or tumoral and infective diseases. The resulting compounds have good bioavailability, increased activity and/or safety.

The parent drugs (i.e. the drugs in which the modification with NF-regulating moiety can be applied) in the present invention can be selected within a wide class of compounds, such as NSAIDs (i.e. unselective cyclo-oxygenase (COX) inhibitors, - ketoprofen, flurbiprofen, suprofen, indobufen, etodolac, indomethacin, naproxen etc.), analgesics (i.e. paracetamol, capsaicin), steroids (chenodeoxycholic acid, flunisolide), antibiotics (bacilysin), bronchodilators (albuterol), expectorants and mucolitic agents (ambroxol), anti-asthma, antiallergic and anti-histaminic drugs (epinastine), ACE-inhibitors (captopril), $\beta$-blockers (atenolol), drugs for vascular disorders (brovincamine), antidiabetics (glibomuride), antitumorals (ancitabine, mopidamol), antiulcer (arbaprostil), antihyperlipidemics (fluvastatine), antibacterials (amoxicilline), antivirals (amantadine), cGMP phosphodiesterase inhibitors (sildenafil, verdenafil), drugs against dementia (mofegiline) and the bone reabsorption (alendronic acid). Also compounds that are nitric oxide donors are useful in the present invention.

When the compounds include at least one asymmetric carbon atom, the products can be used in racemic mixture or in form of single enantiomer.

In the present invention the parent compound is considered in its original form or in a proper modification to allow the chemical manipulation with NF-regulating moieties.

Also parent drugs able to release nitric oxide exogenously or endogenously are useful for the present invention.

NF-regulating moieties include $\alpha$-lipoic acid, $\alpha$-tocoferol, butylated hydroxyanisole (BHA), caffeic acid derivatives, catechol derivatives, cinnamic acid, curcumin, epigallocatechin-3-gallate, ergothioneine, N-(p-aminobenzoyl) glutamic acid, glutathione, hematein, magnolol, nordihydroguaiaretic acid (NDGA), phenolic antioxidants, pyrrolidine dithiocarbamate (PDTC), quercetin, resveratrol, Vitamin C, vitamin E, salicylic acid derivatives (such as cresotic acid isomers and 4-hydroxyisophthalic acid).

Other substances releasing or stimulating the release of hydrogen sulfide and NF-regulating that can be linked to drugs are N-acetyl-penicillamine, S-allyl-cysteine, bucillamine, carbocysteine, cysteamine, cystathionine, homocysteine, mecysteine, methionine, pantetheine, penicillamine, penicillamine disulfide, thioacetic acid, thiodiglycolic acid, thioglycolic acid, thiolactic acid, 2-thiolhistidine, thiomalic acid, thiosalicylic acid, tiopronin. Other substances releasing and/or stimulating the release of hydrogen sulfide and NF-regulating that can be linked to drugs are 5-(p-hydroxyphenyl)-3H-1,2-dithiol-3-thione, 1,3 dithiol-2-thione-5-carboxylic acid, 3-thioxo-3H-1,2-dithiole-5-carboxylic acid, 3-thioxo-3H-1,2-dithiole-4 carboxylic acid.

More groups NF-regulating by releasing $H_2S$ also in combination with groups that release nitric oxide or carbon oxide or that release nitric oxide or carbon oxide per se are part of the present invention.

The substances can be linked via different linking groups such as esters, amides, imides, sulfonamides, azo groups, carbamates, carbonates, anhydrides, acetals, thioacetals, etc.

Bifunctional linkers known to the expert in the field (such as ethyl, propyl, or butyl diols; diamines; hydroxy amines, etc..) can be optionally present when they are necessary to link the drug to the NF-regulating moieties.

Also salts that directly or indirectly are capable to inhibits NF-kB, such as, for example, salts of arginine, agmatine, aminoguanidine, penicillamine, lipoic acid, caffeic acid, 1,3 dithiol-2-thione-5-carboxylic acid, thiosalicylic acid, cinnamic acid etc. are part of the present invention.

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc. A preferred route of administration is the oral route.

The following non-limitative examples further describe and enable an ordinary skilled in the art to make and use the invention.

### EXAMPLE 1. Synthesis of 5-(p-hydroxyphenyl)-3H-1,2-dithiol-3-thione.

To 280 mmol of sulfur 40 mmol of anethole were added. After heating at 200 °C for 6 hours, 2.5 g of anethole dithiolethione were obtained. The product, washed with ether, was crystallized by ethyl acetate: melting point 110-111°C. Then 1.5 g of anethole dithiolethione were mixed with 7.5 g of pyridine HCl and the mixture was heated for 25 minutes at 215°C. After cooling, 1N HCl in excess was added and the precipitate was filtered, washed and crystallized from ethanol. The obtained compound melted at 191-192°C.

### EXAMPLE 2. Synthesis of valproic acid ester with 5-(p-hydroxyphenyl)-3H-1,2-dithiol-3-thione.

The ester of valproic acid with 5-(p-hydroxyphenyl)-3H-1,2-dithiol-3-thione was prepared via the acyl chloride of valproic acid. The acyl chloride of valproic acid (3.0 mmol) and the compound prepared in the example 1 (1.59 mmol) were refluxed in THF anhydrous for 6 hours under nitrogen atmosphere. After removal of THF, the mixture was chromatographed on silica gel eluting with dichloromethane. The compound was crystallized from ethyl ether and showed a melting point of 69.5-70.5°C. Anal. calcd. $C_{17}H_{20}O_2S_3$ C% 57.92; H% 5.72; S% 27.29; found C% 58.05; H% 5.69; S% 27.34.

### EXAMPLE 3. Synthesis of ketoprofen ester with 5-(p-hydroxyphenyl)-3H-1,2-dithiol-3-thione.

The ketoprofen ester was prepared via its acyl chloride and 5-(p-hydroxyphenyl)-3H-1,2-dithiol-3-thione. 2.3 mmol of ketoprofen were suspended in 7 ml of anhydrous THF with 0.92 ml of thionyl chloride, heating at 67°C for 6 hours. After evaporation of THF and thionyl chloride, the ketoprofen acyl chloride was dissolved in anhydrous THF, and a solution of 5-(p-hydroxyphenyl)-3H-1,2-dithiol-3-thione (1.77 mmol.), pyridine (0.2 ml) in THF anhydrous (1 ml) was dropped therein, and the reaction was kept under stirring overnight at room temperature. After evaporation of THF, the product thus obtained was chromatographed on silica gel eluting with dichloromethane. The purified product had a gummy glue-like appearance and its crystallization was obtained adding few drops of ether to the refrigerated compound. The orange crystals showed a melting point of 111.5 - 112.5°C. Anal. calcd. $C_{25}H_{18}O_3S_3$ C% 64.91; H% 3.92; S% 20.79; found C% 64.83; H% 4.02; S% 20.49.

### EXAMPLE 4. Synthesis of ketorolac ester with 5-(p-hydroxyphenyl)-3H-1,2-dithiol-3-thione.

The ketorolac ester was prepared employing dicyclohexylcarbodiimide (DCC) as coupling agent in presence of 4-pyrrolidinylpyridine. The solvent used was methylene chloride purified on basic $Al_2O_3$ to remove traces of ethanol. In a 50 ml flask, 1.17 mmol of ketorolac and 2.15 ml of methylene chloride were charged as well as 0.014 mol of 4-pyrrolidinylpyridine, 1.17 mmol of 5-(p-hydroxyphenyl)-3H-1,2-dithiol-3-thione and 1.47 mmol of DCC (25% excess) in 1.5 ml of methylene chloride. The mixture was stirred for 30 minutes at room temperature. At the end of the reaction, after filtration the solution was extracted with 10% acetic acid, then with 0.1N NaOH and finally with brine. After removal of the solvent, the product was chromatographed on silica gel with dichoromethane and crystallized by ether: melting point 146-147°C. Anal. calcd. $C_{24}H_{17}NO_3S_3$ C% 62.18; H% 3.69; N% 3.02; S% 20.75; found C% 61.85; H% 3.60; N% 2.83; S% 21.10.

### EXAMPLE 5. Synthesis of acetyl thiosalicylic acid 4-(nitroxymethyl)phenyl ester(ATANPE).

To a solution of thiosalicylic acid (39.9 mmol) in KOH (5.6 g in 56 ml of water), 48.9 mmol of acetic anhydride were added under stirring for 1 hour on a ice bath. Adding 4N HC1 a white precipitate was obtained. The solid was filtered and dried.

To a suspension of 5 mmol of the solid in 5 ml of chloroform, 10.3 mmol of oxalyl chloride were added. The mixture was heated at 86 °C and at the end of the reaction the chloroform and oxalyl chloride were evaporated under reduced pressure.

To the acyl chloride solubilized in chloroform, 4.17 mmol of p-hydroxybenzaldehyde and 5 mmol of triethylamine were added. The temperature of the reaction was maintained at 0 °C. The acetyl thiosalicylic p-hydroxybenzaldheide ester was chromatographed on silica gel eluting with cycloexane/ethylacetate (8/2). After crystallization with ether, the compound showed a melting point of 93-94 °C. The ester (1.32 mmol) was transformed in the corresponding p-hydroxybenzyl alcohol with sodium triacetoxy borohydride (5.3 mmoles) in dichloroethane heating under reflux.

The last step was the nitration of the alcohol previously prepared. The mixture for the nitration was constituted by 0.5 ml of fuming $HNO_3$, 1.25 ml of acetic anhydride and 10.75 ml of methylene choride. This mixture (2.7 ml) was added to the alcohol derivative (2.7 mmol in 5 ml of dichloromethane) and the reaction was maintained at 0 °C for 1 hour. After dilution with dichloromethane, the solution was extracted with iced water. The organic phase, dried on anhydrous sodium sulphate, was evaporated. The oily compound, after purification on silica column eluting with dichloromethane and crystallization by ether, had a melting point of 65-66 °C.

## EXAMPLE 6 - Pharmacological

The NF-kB-CAT reporter gene cell line (Lenardo MJ et al.,Cell 58,227-9,1989) and. L29/Ji6 cells (Fratelli M et al, Antioxidants & Redox Signalling 5,229-235,2003) were prepared as previously described. Human TNF alfa (100 ng/ml) was used. The chloramphenicol-acetyltransferase (CAT) activity was measured by adding 40 microliters of Tris-HCl buffer (250 mM, ph 7.5) and D-threo-[dichloroacetyl] 1,2 $^{14}$C chloramphenicol (10 microliters,8.3 x $10^{-2}$ mCi/sample) The reaction was started by the addition of n-butyryl coenzyme A. After 2 h at 37°C, the reaction was stopped by extraction with hexane/xylene (2:1). The upper organic phase containing the reaction product was then recovered, and radioactivity was counted on a beta-counter. The activity on NF-kB was expressed as % of CAT formation.

Table II Effect on NF-kB activity by test compound(100 $\mu$M), as assessed by a reporter gene synthesis: % of CAT synthesis

| Compound | Basal | TNF-stimulated |
|---|---|---|
| Aspirin | 100 | 92 |
| ATANPE | 31 | 26 |

## EXAMPLE 7. Inhibition of the expression of NF-kB regulated cytokine IL-6 and IL-8 in HeLa cells by compound described in example 5.

The cells were stimulated with 100 $\mu$mol of compound described in example 6 with TNF (200 E/ml) or with PMA (50ng/ml)and the concentration of IL-6 or IL-8 was measured in the cell supernatant at various interval using ELISA (British Biotechnology Products Ltd.). The expression values are listed in the following table III.

**TABLE III**

| | IL-6 (pg/ml) | | | IL-8 (pg/ml) | | |
|---|---|---|---|---|---|---|
| | 0' | 15' | 120' | 0' | 15' | 120' |
| TNF | -- | 945 | 7640 | -- | -- | 61 |
| TNF+EX 5 | -- | 945 | 1738 | -- | -- | 6 |
| PMA | -- | 870 | 11813 | -- | -- | 445 |
| PMA+EX 5 | -- | 920 | 3138 | -- | -- | 12 |

The values show that after 120 minutes the compound described in example 5 is able to inhibit the production of IL-6 by 87% or 74% (TNF stimulation) and the production of IL-8 by 90% or 97% (PMA stimulation).

## Claims

1. Compounds able to inhibit the activation of Rel/NF-kB proteins having the formula

$$D-X-Y-S \qquad (I)$$

wherein D is a drug, X is zero or a chemical functional group, such as C=O, COOR, CONH, R being straight or branched $C_1$-$C_4$-alkyl, Y is zero or a bifuntional linker, such as HO-$(CH_2)$n-OH, HOOC-$(CH_2)_n$-COOH, and S is a moiety capable per se or in combination with the drug to modulate the NTFs, with the proviso that when both X and Y are zero, the drug (D) and the moiety (S) are linked by an acid-base bond

$$D^{+/-} . S^{-/+}$$

wherein D is an acid (+) or basic (-) drug and S is a basic (-) or acid (+) compound capable to modulate directly and/or indirectly the NTF, that is a salt of an acid (drug$^-$) with a base (NFs$^+$ modulator) or a salt of a base (drug$^+$) with an acid (NFs-modulator).

2. Compounds according to claim 1, **characterized in that** the drug (D) is selected from the group consisting of NSAIDs, analgesics, antibiotics, bronchodilators, expectorants and mucolitic agents, anti-asthma, antiallergic and anti-histaminic drugs, ACE-inhibitors, β-blockers, drugs for vascular disorders, antidiabetics, antitumorals, antiulcer, antihyperlipidemics, antibacterials, antivirals, cGMP phosphodiesterase inhibitors, steroids, drugs against dementia and bone reabsorption.

3. Compounds according to claim 1, **characterized in that** the moiety S is selected from the group consisting of α-lipoic acid, α-tocoferol, butylated hydroxyanisole (BHA), caffeic acid derivatives, catechol derivatives, cinnamic acid, curcumin, epigallocatechin-3-gallate, ergothioneine, N-(p-aminobenzoyl)glutamic acid, glutathione, hematein, magnolol, nordihydroguaiaretic acid (NDGA), phenolic antioxidants, pyrrolidine dithiocarbamate (PDTC), quercetin, resveratrol, Vitamin C, vitamin E, salicylic acid derivatives (such as cresotic acid isomers and 4-hydroxyisophthalic acid), N-acetyl-penicillamine, S-allyl-cysteine, bucillamine, carbocysteine, cysteamine, cystathionine, homocysteine, mecysteine, methionine, pantetheine, penicillamine, penicillamine disulfide, thioacetic acid, thiodiglycolic acid, thioglycolic acid, thiolactic acid, 2-thiolhistidine, thiomalic acid, thiosalicylic acid, tiopronin, 5-(p-hydroxyphenyl)-3H-1,2-dithiol-3-thione, 1,3 dithiol-2-thione-5-carboxylic acid, 3-thioxo-3H-1,2-dithiole-5-carboxylic acid, 3-thioxo-3H-1,2-dithiole-4 carboxylic acid.

4. Compounds according to claim 1, **characterized in that** the moiety S is selected from groups NF-regulating by releasing $H_2S$ also in combination with groups that release nitric oxide or carbon oxide or that release nitric oxide or carbon oxide.

5. Compounds according to claim 3, **characterized in that** the salcylic acid derivatives are cresotic acid isomers and 4-hydroxyisophthalic acid.

6. Compound according to claim 1, that is valproic acid ester with 5-(p-hydroxyphenyl)-3H-1,2-dithiol-3-thione.

7. Compound according to claim 1, that is ketoprofen ester with 5-(p-hydroxyphenyl)-3H-1,2-dithiol-3-thione.

8. Compound according to claim 1, that is ketorolac ester with 5-(p-hydroxyphenyl)-3H-1,2-dithiol-3-thione.

9. Compound according to claim 1, that is acetylthiosalicylic acid 4-(nitroxymethyl)phenyl ester.

10. Compounds according to claims 1-9 for treating, preventing or reducing oxidative stress associated with cardiovascular, respiratory, connective tissue, nervous, gastrointestinal, tumoral, cutaneous, infective, urogenital diseases.

11. Pharmaceutical composition for treating, preventing or reducing oxidative stress associated with cardiovascular, respiratory, connective tissue, nervous, gastrointestinal, tumoral, cutaneous, infective, urogenital diseases, comprising a compound of formula (I) as active ingredient and a pharmaceutically acceptable adjuvant or carrier.

## PARTIAL EUROPEAN SEARCH REPORT

**European Patent Office**

which under Rule 45 of the European Patent Convention shall be considered, for the purposes of subsequent proceedings, as the European search report

Application Number

EP 04 42 5751

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WAHL C ET AL: "Sulfasalazine: A potent and specific inhibitor of nuclear factor kappa B" JOURNAL OF CLINICAL INVESTIGATION, vol. 101, no. 5, 1 March 1998 (1998-03-01), pages 1163-1174, XP002331540 ISSN: 0021-9738 | 1-4,10, 11 | A61K47/48 A61K31/385 A61K31/407 A61P35/00 A61P9/00 A61P19/04 A61P25/00 A61P1/00 |
| Y | * abstract * <br> * page 1164, left-hand column, paragraph 1 * <br> * page 1167; figure 3 * <br> * page 1169; figure 5 * <br> -----<br> -/-- | 1-5,7-11 | A61P17/00 A61P31/00 A61P13/00 A61P15/00 |

**TECHNICAL FIELDS SEARCHED (Int.Cl.7)**

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 17 June 2005 | Dullaart, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

# PARTIAL EUROPEAN SEARCH REPORT

Application Number

EP 04 42 5751

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | FRATELLI MADDALENA ET AL: "Inhibition of nuclear factor-kappaB by a nitro-derivative of flurbiprofen: a possible mechanism for antiinflammatory and antiproliferative effect." ANTIOXIDANTS & REDOX SIGNALING. APR 2003, vol. 5, no. 2, April 2003 (2003-04), pages 229-235, XP008048117 ISSN: 1523-0864 * abstract * * figures 1,3-6 * * page 229 * | 1-4,10, 11 | |
| Y | EPINAT J-C ET AL: "Diverse agents act at multiple levels to inhibit the Rel/NF-[kappa]B signal transduction pathway" ONCOGENE 22 NOV 1999 UNITED KINGDOM, vol. 18, no. 49, 22 November 1999 (1999-11-22), pages 6896-6909, XP002331541 ISSN: 0950-9232 * abstract * * page 6897; figure 1 * * page 6898 - page 6899; tables 1,2a,2b * | 1-5,7-11 | TECHNICAL FIELDS SEARCHED (Int.Cl.7) |
| X | EP 0 054 936 A (USV PHARMACEUTICAL CORPORATION) 30 June 1982 (1982-06-30) * examples * | 1,3 | |
| X | WO 98/40056 A (SMITHKLINE BEECHAM PLC) 17 September 1998 (1998-09-17) * page 26 * * claim 13 * | 1-4,10, 11 | |

-/--

EPO FORM 1503 03.82 (P04C10)

**European Patent Office**

## PARTIAL EUROPEAN SEARCH REPORT

Application Number

EP 04 42 5751

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | WO 98/20864 A (UNIVERSITA' DEGLI STUDI DI BRESCIA - DIPARTIMENTO; GRILLI, MARIAGRAZIA) 22 May 1998 (1998-05-22) * page 12 * ----- | 1-5,7-11 | |
| X | EP 1 352 650 A (INSTITUTE OF MEDICINAL MOLECULAR DESIGN, INC) 15 October 2003 (2003-10-15) * examples * ----- | 1-4,10, 11 | |
| E | EP 1 510 210 A (INSTITUTE OF MEDICINAL MOLECULAR DESIGN, INC) 2 March 2005 (2005-03-02) * examples * ----- | 1-4,10, 11 | TECHNICAL FIELDS SEARCHED (Int.Cl.7) |
| E | EP 1 512 397 A (INSTITUTE OF MEDICINAL MOLECULAR DESIGN, INC) 9 March 2005 (2005-03-09) * examples * ----- | 1-4,10, 11 | |
| Y | LAM S ET AL: "A phase II clinical trial of anethole dithiolethione (Sialor(R), Sulfarlem(R)) in smokers with bronchial dysplasia" EUROPEAN JOURNAL OF CANCER, PERGAMON PRESS, OXFORD, GB, vol. 38, February 2002 (2002-02), page S45, ABSTRACT P16, XP004367808 ISSN: 0959-8049 * the whole document * ----- -/-- | 1-5,7,8, 10,11 | |

EPO FORM 1503 03.82 (P04C10)

**European Patent Office**

## PARTIAL EUROPEAN SEARCH REPORT

Application Number

EP 04 42 5751

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | LUBET R A ET AL: "Chemopreventive efficacy of anethole trithione, N-acetyl-L-cysteine, miconazole and phenethylisothiocyanate in the DMBA-induced rat mammary cancer model" INTERNATIONAL JOURNAL OF CANCER, NEW YORK, NY, US, vol. 72, no. 1, 1997, pages 95-101, XP002237061 ISSN: 0020-7136 * abstract * * figures 1,5 * * table 1 * * page 98, paragraph DISCUSSION - page 100 * | 1-5,7,8, 10,11 | |
| Y | REDDY B S ET AL: "CHEMOPREVENTION OF COLON CARCINOGENESIS BY ORGANOSULFUR COMPOUNDS" CANCER RESEARCH, AMERICAN ASSOCIATION FOR CANCER RESEARCH, BALTIMORE, MD, US, vol. 53, no. 15, 1 August 1993 (1993-08-01), pages 3493-3498, XP001097689 ISSN: 0008-5472 * abstract * * figure 1 * * tables 1,2 * | 1-5,7-11 | **TECHNICAL FIELDS SEARCHED** (Int.Cl.7) |
| Y | EP 0 236 929 A (MITSUI TOATSU CHEMICALS INC) 16 September 1987 (1987-09-16) * examples * | 1-5,7,8, 10,11 | |

-/--

EPO FORM 1503 03.82 (P04C10)

European Patent
Office

**PARTIAL EUROPEAN SEARCH REPORT**

Application Number

EP 04 42 5751

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | PATENT ABSTRACTS OF JAPAN vol. 1995, no. 08, 29 September 1995 (1995-09-29) & JP 07 112978 A (MITSUI TOATSU CHEM INC), 2 May 1995 (1995-05-02) * abstract * | 1-5,7,8, 10,11 | |
| Y | DRUKARCH B ET AL: "ANETHOLE DITHIOLETHIONE PREVENTS OXIDATIVE DAMAGE IN GLUTATHIONE- DEPLETED ASTROCYTES" EUROPEAN JOURNAL OF PHARMACOLOGY, AMSTERDAM, NL, vol. 329, no. 2/3, 25 June 1997 (1997-06-25), pages 259-262, XP000981645 ISSN: 0014-2999 * abstract * * page 260, paragraph RESULTS - page 261 * | 1-5,7,8, 10,11 | |
| X | US 2002/068098 A1 (BABISH JOHN G ET AL) 6 June 2002 (2002-06-06) * examples 1-3 * | 1-4 | TECHNICAL FIELDS SEARCHED (Int.Cl.7) |
| Y | WO 01/62085 A (MEDINOX, INC) 30 August 2001 (2001-08-30) * page 7 * | 1-4,10, 11 | |
| X | DE 199 35 302 A1 (AVENTIS PHARMA DEUTSCHLAND GMBH) 8 February 2001 (2001-02-08) * page 4, column 28 * * examples * * tables * | 1-4,10, 11 | |
| E | EP 1 535 609 A (INSTITUTE OF MEDICINAL MOLECULAR DESIGN, INC) 1 June 2005 (2005-06-01) * examples * | 1-3,10, 11 | |

-/--

EPO FORM 1503 03.82 (P04C10)

European Patent
Office

**PARTIAL EUROPEAN SEARCH REPORT**

Application Number

EP 04 42 5751

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | EP 1 364 943 A (AJINOMOTO CO., INC) 26 November 2003 (2003-11-26) * examples * | 1-3,10, 11 | |
| Y | SHIOJIRI T ET AL: "PPAR[gamma] ligands inhibit nitrotyrosine formation and inflammatory mediator expressions in adjuvant-induced rheumatoid arthritis mice" EUROPEAN JOURNAL OF PHARMACOLOGY 19 JUL 2002 NETHERLANDS, vol. 448, no. 2-3, 19 July 2002 (2002-07-19), pages 231-238, XP001199447 ISSN: 0014-2999 * abstract * * page 237 * | 1-4,10, 11 | |
| Y | ANDREW P J ET AL: "Nitric oxide regulates IL-8 expression in melanoma cells at the transcriptional level" BIOCHEMICAL AND BIOPHYSICAL RESEARCH COMMUNICATIONS 1995 UNITED STATES, vol. 214, no. 3, 1995, pages 949-956, XP001148778 ISSN: 0006-291X * abstract * * page 953 - page 955 * | 1-4,10, 11 | |

TECHNICAL FIELDS
SEARCHED (Int.Cl.7)

European Patent
Office

INCOMPLETE SEARCH
SHEET C

Application Number

EP 04 42 5751

Claim(s) searched completely:
7-9

Claim(s) searched incompletely:
1-5, 10, 11

Reason for the limitation of the search:

Claims 1-5, 10 and 11 encompass a genus of compounds defined only by their function, or by the function of certan chemical groups these compounds contain, wherein the relationship between the structural features of the members of the genus and said function have not been defined. In the absence of such a relationship either disclosed in the as-filed application or which would have been recognized based upon information readily available to one skilled in the art, the skilled artisan would not know how to make and use compounds that lack structural definition. The fact that one could have assayed a compound of interest using the claimed assays does not overcome this defect since one would have no knowledge beforehand as to whether or not any given compound (other than those that might be particularly disclosed in an application) would fall within the scope of what is claimed. It would require undue experimentation (be an undue burden) to randomly screen undefined compounds for the claimed activity. Therefore, only a partial search has been performed for claims 1-5, 10 and 11 (Art. 83 and Art. 84 EPC). Moreover, present claims 1-5, 10 and 11 relate to a compound defined by reference to a desirable characteristic or property, namely its ability to modulate nuclear transcription factors (NTFs).
The claims cover all compounds having this characteristic or property, whereas the application provides support within the meaning of Article 84 EPC and/or disclosure within the meaning of Article 83 EPC for only a very limited number of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the product/compound/method/apparatus by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the compounds prepared in the examples, or specifically mentioned in the claims, within the limits of the first invention as defined under the objection for lack of unity of invention.

European Patent
Office

Application Number

EP 04 42 5751

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5, 10 and 11 in part, and 7-9

**LACK OF UNITY OF INVENTION
SHEET B**

The Search Division considers that the present European patent application does not comply with the requirements of unity of invention and relates to several inventions or groups of inventions, namely:

1. claims: 1-5, 10 and 11 in part, and 7-9

    Compound of the formula D-X-Y-S as defined in these claims, wherein S is a compound according to claim 3, and the drug D is an NSAID.

    ---

2. claims: 1-5, 10 and 11 in part

    Compound of the formula D-X-Y-S as defined in these claims, wherein S is a compound according to claim 3, and the drug D is an analgesic, not being an NSAID.

    ---

3. claims: 1-5, 10 and 11 in part

    Compound of the formula D-X-Y-S as defined in these claims, wherein S is a compound according to claim 3, and the drug D is an antibiotic or an antibacterial drug.

    ---

4. claims: 1-5, 10 and 11 in part

    Compound of the formula D-X-Y-S as defined in these claims, wherein S is a compound according to claim 3, and the drug D is a bronchodilator.

    ---

5. claims: 1-5, 10 and 11 in part

    Compound of the formula D-X-Y-S as defined in these claims, wherein S is a compound according to claim 3, and the drug D is an expectorant.

    ---

6. claims: 1-5, 10 and 11 in part

    Compound of the formula D-X-Y-S as defined in these claims, wherein S is a compound according to claim 3, and the drug D is a mucolytic agent.

    ---

7. claims: 1-5, 10 and 11 in part

    Compound of the formula D-X-Y-S as defined in these claims, wherein S is a compound according to claim 3, and the drug D is an anti-asthma or antiallergic drug, more specifically an antihistaminic drug.

    ---

8. claims: 1-5, 10 and 11 in part

European Patent
Office

**LACK OF UNITY OF INVENTION
SHEET B**

Application Number

EP 04 42 5751

The Search Division considers that the present European patent application does not comply with the requirements of unity of invention and relates to several inventions or groups of inventions, namely:

Compound of the formula D-X-Y-S as defined in these claims, wherein S is a compound according to claim 3, and the drug D is an ACE-inhibitor.

---

9.  claims: 1-5, 10 and 11 in part

Compound of the formula D-X-Y-S as defined in these claims, wherein S is a compound according to claim 3, and the drug D is a beta blocker.

---

10. claims: 1-5, 10 and 11 in part

Compound of the formula D-X-Y-S as defined in these claims, wherein S is a compound according to claim 3, and the drug D is a drug for vascular disorders, not being an ACE inhibitor or a beta blocker.

---

11. claims: 1-5, 10 and 11 in part

Compound of the formula D-X-Y-S as defined in these claims, wherein S is a compound according to claim 3, and the drug D is an antidiabetic.

---

12. claims: 1-5, 10 and 11 in part

Compound of the formula D-X-Y-S as defined in these claims, wherein S is a compound according to claim 3, and the drug D is an antitumour drug.

---

13. claims: 1-5, 10 and 11 in part

Compound of the formula D-X-Y-S as defined in these claims, wherein S is a compound according to claim 3, and the drug D is an antiulcer drug.

---

14. claims: 1-5, 10 and 11 in part

Compound of the formula D-X-Y-S as defined in these claims, wherein S is a compound according to claim 3, and the drug D is an antihyperlipidemic drug.

---

15. claims: 1-5, 10 and 11 in part

European Patent
Office

**LACK OF UNITY OF INVENTION
SHEET B**

Application Number

EP 04 42 5751

The Search Division considers that the present European patent application does not comply with the requirements of unity of invention and relates to several inventions or groups of inventions, namely:

Compound of the formula D-X-Y-S as defined in these claims, wherein S is a compound according to claim 3, and the drug D is an antiviral drug.

---

16. claims: 1-5, 10 and 11 in part

Compound of the formula D-X-Y-S as defined in these claims, wherein S is a compound according to claim 3, and the drug D is a cGMP phosphodiesterase inhibitor.

---

17. claims: 1-5, 10 and 11 in part

Compound of the formula D-X-Y-S as defined in these claims, wherein S is a compound according to claim 3, and the drug D is a steroid.

---

18. claims: 1-5, 10 and 11 in part

Compound of the formula D-X-Y-S as defined in these claims, wherein S is a compound according to claim 3, and the drug D is a drug against dementia.

---

19. claims: 1-5, 10 and 11 in part

Compound of the formula D-X-Y-S as defined in these claims, wherein S is a compound according to claim 3, and the drug D is a drug against bone reabsorption.

---

20. claims: 1-5, 10 and 11 in part, and 6

Valproic acid ester with 5-(p-hydroxyphenyl)-3H-1,2-dithioil-3-thione.

---

## ANNEX TO THE EUROPEAN SEARCH REPORT
## ON EUROPEAN PATENT APPLICATION NO.

EP 04 42 5751

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2005

| Patent document cited in search report | | Publication date | Patent family member(s) | | Publication date |
|---|---|---|---|---|---|
| EP 0054936 | A | 30-06-1982 | AU | 546887 B2 | 26-09-1985 |
| | | | AU | 7876181 A | 01-07-1982 |
| | | | CA | 1191139 A1 | 30-07-1985 |
| | | | DE | 3174787 D1 | 10-07-1986 |
| | | | ES | 8300318 A1 | 16-01-1983 |
| | | | IE | 52164 B1 | 22-07-1987 |
| | | | IL | 64616 A | 29-11-1985 |
| | | | JP | 2031707 B | 16-07-1990 |
| | | | JP | 57131749 A | 14-08-1982 |
| | | | NZ | 199358 A | 30-04-1985 |
| | | | US | 4440941 A | 03-04-1984 |
| | | | ZA | 8108870 A | 24-11-1982 |
| WO 9840056 | A | 17-09-1998 | CA | 2285446 A1 | 17-09-1998 |
| | | | EP | 0970062 A2 | 12-01-2000 |
| | | | JP | 2001524074 T | 27-11-2001 |
| | | | US | 6211212 B1 | 03-04-2001 |
| WO 9820864 | A | 22-05-1998 | IT | MI962356 A1 | 13-05-1998 |
| EP 1352650 | A | 15-10-2003 | AU | 2268302 A | 01-07-2002 |
| | | | CA | 2431083 A1 | 27-06-2002 |
| | | | CN | 1489458 A | 14-04-2004 |
| | | | WO | 0249632 A1 | 27-06-2002 |
| | | | US | 2004259877 A1 | 23-12-2004 |
| EP 1510210 | A | 02-03-2005 | AU | 2003242131 A1 | 22-12-2003 |
| | | | CA | 2487900 A1 | 18-12-2003 |
| | | | WO | 03103658 A1 | 18-12-2003 |
| EP 1512397 | A | 09-03-2005 | AU | 2003242118 A1 | 22-12-2003 |
| | | | CA | 2488363 A1 | 18-12-2003 |
| | | | WO | 03103656 A1 | 18-12-2003 |
| EP 0236929 | A | 16-09-1987 | CA | 1301177 C | 19-05-1992 |
| | | | DE | 3767894 D1 | 14-03-1991 |
| | | | HU | 47096 A2 | 30-01-1989 |
| | | | US | 4760078 A | 26-07-1988 |
| JP 07112978 | A | 02-05-1995 | NONE | | |
| US 2002068098 | A1 | 06-06-2002 | NONE | | |
| WO 0162085 | A | 30-08-2001 | AU | 4172901 A | 03-09-2001 |
| | | | US | 6710086 B1 | 23-03-2004 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 04 42 5751

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2005

| Patent document cited in search report | | Publication date | Patent family member(s) | | Publication date |
|---|---|---|---|---|---|
| DE 19935302 | A1 | 08-02-2001 | AU | 776114 B2 | 26-08-2004 |
| | | | AU | 6825200 A | 19-02-2001 |
| | | | BR | 0012757 A | 02-04-2002 |
| | | | CA | 2377977 A1 | 08-02-2001 |
| | | | CN | 1360507 A | 24-07-2002 |
| | | | CZ | 20020300 A3 | 15-05-2002 |
| | | | EE | 200200035 A | 15-04-2003 |
| | | | WO | 0108707 A2 | 08-02-2001 |
| | | | EP | 1204430 A2 | 15-05-2002 |
| | | | HU | 0201995 A2 | 28-10-2002 |
| | | | JP | 2003505517 T | 12-02-2003 |
| | | | MX | PA01013114 A | 04-06-2002 |
| | | | NO | 20020367 A | 26-03-2002 |
| | | | NZ | 516838 A | 30-07-2004 |
| | | | PL | 353069 A1 | 06-10-2003 |
| | | | SK | 1172002 A3 | 06-08-2002 |
| | | | TR | 200200222 T2 | 22-07-2002 |
| | | | ZA | 200200657 A | 25-08-2003 |
| EP 1535609 | A | 01-06-2005 | AU | 2003242098 A1 | 22-12-2003 |
| | | | CA | 2489091 A1 | 18-12-2003 |
| | | | WO | 03103654 A1 | 18-12-2003 |
| EP 1364943 | A | 26-11-2003 | WO | 02062751 A1 | 15-08-2002 |
| | | | JP | 2002226457 A | 14-08-2002 |
| | | | US | 2004059110 A1 | 25-03-2004 |

EPO FORM P0459

For more details about this annex : see Official Journal of the European Patent Office, No. 12/82